# EUROPEAN PATENT APPLICATION

(11) **EP 2 873 440 A1**
(43) Date of publication of application: **20.05.2015**
(21) Application number: 13193399.6
(22) Date of filing: 19.11.2013
(51) Int. Cl.: A61Q 7/00, A61K 8/97

(54) **Use of an edelweiss extract in hair care for stimulating hair growth**

(71) Applicant: DSM IP Assets B.V., 6411 TE Heerlen (NL)
(72) Inventor: The designation of the inventor has not yet been filed
(74) Representative: Barthelemy de Saizieu, Antoine

(57) **Abstract**

The present invention relates to a new use of an edelweiss extract for application to skin, skin having hair, scalp, hair of a human, for lessening hair loss, restoring hair growth after the onset of baldness has occurred, increasing the thickness of hair, counteracting age-associated hair thinning, preventing premature hair loss, or delaying the onset or severity of age-associated hair loss and thinning. This invention also relates to a method for stimulating hair growth.

## Description

The present invention relates to a new use of an edelweiss extract for application to skin, skin having hair, scalp, hair of a human, for lessening hair loss, restoring hair growth after the onset of baldness has occurred, increasing the thickness of hair, counteracting age-associated hair thinning, preventing premature hair loss, or delaying the onset or severity of age-associated hair loss and thinning. This invention also relates to a method for stimulating hair growth.

### BACKGROUND OF THE INVENTION

The hair is composed of a protein called keratin. The hair itself is arranged in three layers, an outer cuticle, middle cortex and central medulla. Hair grows from a follicle. The walls of the follicle form the outer root sheath of the hair. The lower part of the follicle widens out to form the hair bulb that contains the germinal matrix, the source of hair growth. Dermal tissue projects into the follicle base to form the dermal papilla, and this has a network of capillary blood vessels to supply oxygen, energy, and the amino-acids needed for hair growth.

Correcting the effects of aging as far as possible is a preoccupation of ever-increasing importance. It therefore remains a long awaited need in the hair care industry to prevent hair loss, to stimulate hair growth, and to increase hair thickness in order to improve facial appearance of an individual.

There is still a need to find compositions having a beneficial effect on hair growth in human being of natural origin, and being safe in use.

### DETAILED DESCRIPTION OF THE INVENTION

The inventors of the present application now surprisingly found that an edelweiss extract has a great potential for use in topical hair care applications for lessening hair loss, restoring hair growth after the onset of baldness has occurred, increasing the thickness of hair, counteracting age-associated hair thinning, preventing premature hair loss, or delaying the onset or severity of age-associated hair loss and thinning.

Thus in a first embodiment, the present invention provides the use of a topical composition comprising an edelweiss extract for lessening hair loss, restoring hair growth after the onset of baldness has occurred, increasing the thickness of hair, counteracting age-associated hair thinning, preventing premature hair loss, or delaying the onset or severity of age-associated hair loss and thinning.

In a further embodiment, the present invention provides a method for stimulating hair growth, comprising the steps of applying to skin having hair, for a sufficient time, a topical composition comprising an effective amount of an edelweiss extract, and observing the results.

The present invention further provides a topical composition comprising an edelweiss extract for use in lessening hair loss, restoring hair growth after the onset of baldness has occurred, increasing the thickness of hair, counteracting age-associated hair thinning, preventing premature hair loss, or delaying the onset or severity of age-associated hair loss and thinning.

The edelweiss extract used for the present invention is derived from the aerial parts (flowers) of *Leontopodium alpinum* Cass. *Leontopodium alpinum* Cass. (edelweiss) is one of the most famous plants in the European Alps and grows in high altitude. Typically, such an extract is rich in phenolic acids, Leontopodic acid (a highly substituted hexaric acid derivative), which is well known to have powerful antioxidant properties. It is also rich in other phenolic acids (chlorogenic acid), flavonoids (luteolin-4'-O-glucoside, apigenin-7-O-glucoside, luteolin), and tannin. In a preferred embodiment, the extract is as described in (WO2001/087256) and is commercialized under the brand name of ALPAFLOR^{®} EDELWEISS by DSM Nutritional Products.

Typically, the edelweiss extract is prepared as follows: Aerial parts of Leontopodium alpinum are dried under hot air flow, followed by milling. The dried plants are then extracted with an ethanol/water solution. The ethanol is then removed by vacuum distillation, and the concentrate is diluted with glycerin, or a glycerin/ethanol mixture.

The compositions according to the invention are especially attractive, since many people, including animal owners and handlers, have a special interest in cosmetic treatments considered as "natural" with mild effects and without major side effects.

As used throughout the specification and claims, the following definitions apply:
The term "Hair" as used according to the invention refers to both, hair of a human being as well as animal fur.

The term "skin having hair" relates to all parts of the skin of a human having hair such as in particular the scalp and the face (eyelashes, the eyebrows, beard). Most preferably the topical compositions are applied to the scalp of humans (male or female of any age).

"Topical composition" as used herein denotes any composition suitable for the topical application to mammalian keratinous tissue such as skin having hair, particularly to the human scalp or to animal skin having fur.

"Preventing" as used herein is not intended to mean that the event will never occur, but means delaying the onset of the condition or event, and/or lessening the severity of the condition or event when it does occur.

"applying to skin" is meant to convey that application of the active ingredient regularly occurs over an extended period of time, for example once or twice per day for a time of at least about two weeks, preferably for at least one month, and more preferably at least two months. Alternatively, the regular application can be every two days, every three days, or once per week or twice per week.

"Sufficient time" means substantially daily for a period of time of at least about two weeks, preferably at least about a month, and even more preferably for at least about two months.

"Observing" may be done by either the individual who applies the active ingredient topically, or may be done by a third party. The post-administration condition may be compared with the pre-administration condition and analyzed either using a standard test, or by subjective analysis.

The edelweiss extract according to the present invention may either be used in therapeutic or non-therapeutic topical applications.

In a preferred embodiment, the use according to the present invention is non-therapeutic.

In a preferred embodiment, the edelweiss extract is combined with at least one additional active substance selected from the group consisting of: antioxidants, light screening agents, and colorants.

### Antioxidants

Based on the invention all known antioxidants usually formulated into hair care compositions can be used. Especially preferred are antioxidants chosen from the group consisting of amino acids (e.g. glycine, histidine, tyrosine, tryptophan) and their derivatives, imidazole (e.g. urocanic acid) and derivatives, peptides such as D,L-carnosine, D-carnosine, L-carnosine and derivatives (e.g. anserine), carotenoids, carotenes (e.g. α-carotene, β-carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cystine, cystamine and its glycosyl-, N-acetyl-, methyl-, ethyl-, propyl-, amyl-, butyl- and lauryl-, palmitoyl-; oleyl-, y-linoleyl-, cholesteryl- and glycerylester) and the salts thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinesulfoximine, buthioninsulfone, penta-, hexa-, heptathioninsulfoximine) in very low compatible doses (e.g. pmol to µmol/ kg), additionally (metal)-chelators (such as α-hydroxyfatty acids (citric acid, lactic acid, malic acid), palmic-, phytinic acid, , lactoferrin), β-hydroxyacids, huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, tocopherol and derivates (such as vitamin-E-acetate), mixtures of nat. vitamin E, vitamin A and derivatives (vitamin-A-palmitate and -acetate) as well as coniferylbenzoate, rutinic acid and derivatives, α-glycosylrutin, ferulic acid, furfurylideneglucitol, carnosine, butylhydroxytoluene, butylhydroxyanisole, trihydroxybutyrophenone, urea and its derivatives, mannose and derivatives, zinc and derivatives (e.g. ZnO, ZnSO₄), selen and derivatives (e.g. selenomethionin), stilbenes and derivatives (such as stilbenoxide, trans-stilbenoxide) and suitable derivatives (salts, esters, ethers, sugars, nucleotides, nucleosides, peptides and lipids) of the named active ingredients, or enzymes such as superoxide dismutase, catalase or similar, or activators of such enzymes. One or more preservatives/ antioxidants may be present in an amount of at least 0.01 wt.-% of the total weight of the composition. Preferably about 0.01 to about 10 wt.-% of the total weight of the composition of the present invention is present. Most preferred, one or more preservatives/ antioxidants are present in an amount about 0.1 to about 1 wt. -%.

### Light screening agents

Light screening agents are advantageously selected from UV-A, UV-B and/or broadband filters. Examples of UV-B or broad spectrum screening agents, i.e. substances having absorption maximums between about 290 and 340 nm may be organic or inorganic compounds. Organic UV-B or broadband screening agents are e.g. acrylates such as 2-ethylhexyl 2-cyano-3,3-diphenylacrylate (octocrylene, PARSOL^{®} 340), ethyl 2-cyano-3,3-diphenylacrylate and the like; camphor derivatives such as 4-methyl benzylidene camphor (PARSOL^{®} 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like; Cinnamate derivatives such as ethylhexyl methoxycinnamate (PARSOL^{®} MCX), ethoxyethyl methoxycinnamate, diethanolamine methoxycinnamate (PARSOL^{®} Hydro), isoamyl methoxycinnamate and the like as well as cinnamic acid derivatives bond to siloxanes; p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate; benzophenones such as benzophenone-3, benzophenone-4,2,2',4,4'-tetrahydroxy-benzophenone, 2,2'-dihydroxy-4,4'-dimethoxybenzophenone and the like; esters of benzalmalonic acid such as di-(2-ethylhexyl) 4-methoxybenzalmalonate; esters of 2-(4-ethoxy-anilinomethylene)propandioic acid such as 2-(4-ethoxy anilinomethylene) propandioic acid diethyl ester as described in the European Patent Publication EP 0895 776; organosiloxane compounds containing benzmalonate groups as described in the European Patent Publications EP 0 358 584 B1, EP 0 538 431 B1 and EP 0 709 080 A1 such as polysilicone-15 (PARSOL^{®} SLX); drometrizole trisiloxane (Mexoryl XL); imidazole derivatives such as e.g. 2-phenyl benzimidazole sulfonic acid and its salts (PARSOL^{®}HS). Salts of 2-phenyl benzimidazole sulfonic acid are e.g. alkali salts such as sodium- or potassium salts, ammonium salts, morpholine salts, salts of prim., sec. and tert. amines like monoethanol amine salts, diethanol amine salts and the like; salicylate derivatives such as isopropylbenzyl salicylate, benzyl salicylate, butyl salicylate, ethylhexyl salicylate (PARSOL^{®} EHS, NEO Heliopan OS), isooctyl salicylate or homomenthyl salicylate (homosalate, PARSOL^{®} HMS, NEO Heliopan OS) and the like; triazine derivatives such as ethylhexyl triazone (Uvinul T-150), diethylhexyl butamido triazone (Uvasorb HEB). Encapsulated UV-filters such as encapsulated ethylhexyl methoxycinnamate (Eusolex UV-pearls) or microcapsules loaded with UV-filters as e.g. disclosed in EP 1 471 995 and the like. Inorganic compounds are pigments such as microparticulated TiO₂, ZnO and the like. The term "microparticulated" refers to a particle size from about 5 nm to about 200 nm, particularly from about 15 nm to about 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.

Examples of broad spectrum or UV A screening agents i.e. substances having absorption maximums between about 320 and 400 nm may be organic or inorganic compounds e.g. dibenzoylmethane derivatives such as 4-tert. butyl-4'-methoxydibenzoyl-methane (PARSOL^{®} 1789), dimethoxydibenzoylmethane, isopropyldibenzoylmethane and the like; benzotriazole derivatives such as 2,2'-methylene-bis-(6-(2H-benzotriazole-2-yl)-4-(1,1,3,3,-tetramethylbutyl)-phenol (TINOSORB M) and the like; bis-ethylhexyloxyphenol methoxyphenyl triazine (Tinosorb S) and the like; phenylene-1,4-bis-benzimidazolsulfonic acids or salts such as 2,2-(1,4-phenylene)bis-(1H-benzimidazol-4,6-disulfonic acid) (Neoheliopan AP); amino substituted hydroxybenzophenones such as 2-(4-diethylamino-2-hydroxy-benzoyl)-benzoic acid hexylester (Uvinul A plus) as described in EP1046391; Ionic UV-A filters as described in WO 2005/080341 A1; pigments such as microparticulated ZnO or TiO₂ and the like. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art. As dibenzoylmethane derivatives have limited photostability it may be desirable to photostabilize these UV-A screening agents. Thus, the term "conventional UV-A screening agent" also refers to dibenzoylmethane derivatives such as e.g. PARSOL^{®} 1789 stabilized by, e.g. 3,3-Diphenylacrylate derivatives as described in EP0514491 B1 and EP0780119 A1; Benzylidene camphor derivatives as described in the US Patent No. 5,605,680; Organosiloxanes containing benzmalonate groups as described in the EP0358584 B1, EP0538431 B1 and EP0709080 A1.

### Colorants

Based on the invention, all colorants usually formulated into hair care compositions which have an absorption in the visible light of electromagnetic radiation (400 nm to 800 nm) can be used. The absorption is often caused by the following chromophores: Azo- (mono-, di-, tris-, or poly-)stilbene-, carotenoide-, diarylmethane-, triarylmethane-, xanthene-, acridine-, quinoline-, methine- (also polymethine-) thiazol-, indamine-, indophenol-, azin-, oxazine-, thiazine-, anthraquinone-, indigo-, phthalocyanin and further synthetic, natural and/or inorganic chromophores.

FD & C and D & C which can be used in hair care compositions according to the invention are e.g. curcumin, riboflavin, lactoflavin, tartrazine, quinoline yellow, cochenille, azorubin, amaranth, ponceau 4R, erythrosine, red 2G, indigotin, chlorophyll, chlorophyllin, caramel, carbo medicinalis, carotenoids, carotin, bixin, norbixin, annatto, orlean, capsanthin, capsorubin, lycopin, xanthophyll, flavoxanthin, lutein, kryptoaxanthin, rubixanthin, violaxanthin, rhodoxanthin, canthaxanthin, betanin, anthocyans without being limited thereto. Examples of dyes are e.g. inorganic pigments such as iron oxide (iron oxide red, iron oxide yellow, iron oxide black etc.) ultramarines, chromium oxide green or carbon black. Other colorants and dyes which can be used in the compositions according to the invention comprise natural or synthetic organic pigments, disperse dyes which may be solubilized in solvents like direct hair dyes of the HC type, for example HC red No. 3, HC Blue No. 2 and all other hair dyes listed in International Cosmetic Ingredient Dictionary Handbook, 11th edition, 2006) or the dispersion dyes listed in Color Index International Society of Dyers and Colorist, color varnishes (insoluble salts of soluble dyes, like many Ca-, Ba- or Al-salts of anionic dyes), soluble anionic or cationic dyes such as acid dyes (anionic), basic dyes (cationic), direct dyes, reactive dyes or solvent dyes, fluorescent dyes, fluorescein and isothiocyanates.

In yet another embodiment, the edelweiss extract of the present invention is used topically on hair, wherein hair is a mammal's fur. Preferred mammals are horses, dogs and cats.

In a further embodiment, the edelweiss extract is used in an effective amount selected in the range of 0.00001 to 20 weight-%, preferably, 0.0001 to 10 weight-%, more preferably, 0.001 to 1 weight-% based on the total weight of the topical composition.

Preferably, the topical composition for the method and use according to the present invention is a hair care composition, and more preferably, it is a hair tonic, a conditioner, a shampoo, or a styling gel. The topical composition may further comprise at least one additional active substance selected from the group consisting of antioxidants, light screening agents, and colorants.

### Hair Loss

Also according to this invention, topical applications of an edelweiss extract can:
○ lessening hair loss,
○ restoring hair growth after the onset of baldness has occurred,
○ increasing the thickness of hair,
○ counteracting age-associated hair thinning,
○ preventing or counteracting premature hair loss,
○ delaying the onset or severity of age-associated hair loss, and/or
○ delaying the onset or severity of hair loss and thinning.

Thus another aspect of this invention is a method of stimulating hair growth, comprising the steps of applying to skin having hair, for a sufficient time, a topical composition, preferably, a hair care composition, comprising an effective amount of an edelweiss extract, and observing the results.

The present invention also relates to the use of steviol and/or isosteviol or a salt, an ester, a diester, or an ether thereof as depicted in formula (I) for stimulating dermal papilla region and keratinocytes within the hair follicle in order to increase the total number of keratinocytes responsible for hair growth and/or releasing growth stimulating molecular signals from surrounding cells of the hair follicle and/or migration of progenitor keratinocytes to rebuild hair follicles.

In particular the topical compositions are hair care compositions such as conditioners, treatments, hair tonics, styling gels, mousses, shampoos, hair sprays, pomades, setting lotions, coloring and permanent waving compositions. Of particular interest for the purpose of the present invention are tonics, conditioners, treatments, and styling gels which may be in the form of a gel, a lotion, a tincture, a spray, a mousse, a cleansing composition or a foam and which may be applied according to individual needs, e.g., once daily as a lotion, tincture, mousse or spray; or once or twice weekly as a conditioner or treatment.

The edelweiss extract according to the present invention may be incorporated into conventional hair care compositions as described below:
The hair care compositions may comprise additional cosmetic or dermatological adjuvants and/or additives (cosmetic carrier) which are preferably selected from
   1.) Water
   2.) Water soluble organic solvents, preferably C1-C4-Alkanols
   3.) Oils, fatty substances, waxes
   4.) Various esters different to 3) of C6-C30 monocarboxylic acids with mono-, di- or trivalent alcohols
   5.) Saturated acyclic and cyclic hydrocarbons
   6.) Fatty acids
   7.) Fatty alcohols
   8.) Silicone oils
   9.) Surface active ingredients
and mixtures thereof.

The hair care compositions can contain further adjuvants and additives such as preservatives, antioxidants, silicones, thickeners, softeners, anionic, cationic, nonionic or amphoteric emulsifiers, light screening agents, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, light stabilizers, insect repellents, antibacterial agents, or any other ingredients usually formulated into hair care compositions. The necessary amounts of the adjuvants and additives can, based on the desired product, easily be chosen by a skilled artisan in this field and will be illustrated in the examples, without being limited hereto.

Preferably the hair care compositions are in the form of cosmetic hair-treatment preparations, e.g. hair tonics, conditioners, hair-care preparations, e.g. pre-treatment preparations, styling creams, styling gels, pomades, hair rinses, treatment packs, intensive hair treatments e.g. leave-on and rinse-off deep conditioners, hair-structuring preparations, e.g. hair-waving preparations for permanent waves (hot wave, mild wave, cold wave), hair-straightening preparations, liquid hair-setting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colorants, preparations containing self-oxidizing dyes, or natural hair colorants, such as henna or chamomile.

Preferred hair care compositions are leave-on compositions selected from hair tonics, conditioners, treatments, and styling gels.

Based on the application the hair care preparations may be in the form of a (aerosol) spray, (aerosol) foam, gel, gel spray, cream, lotion, liquid or a wax. Hair sprays comprise as well aerosol sprays as pump sprays without propellant. Hair foams comprise as well aerosol foams as pump foams without propellant. Hair sprays and hair foams comprise mainly or exclusively water soluble or water dispersible components. If the components used in hair sprays or hair foams according to the invention are water dispersible, then they may be in the form of micro dispersions with particle sizes of usually 1-350 nm, preferably 1-250 nm. The solid content of such preparations is typically in the range of 0.5 to 20 wt.-% of the total weight of the preparation. Such micro dispersions normally do not need further emulsifiers or tensides for their stabilization.

An exemplary hair gel with the compound of the present invention may comprise:
1. 0.1 to 20 wt.-% preferably 1 to 10 wt.-% of at least one hair polymer;
2. 0 to 10 wt.-% of at least one carrier (solvent), selected from C2-C5 alcohols, preferably ethanol;
3. 0.01 to 5 wt.-%, preferably 0.2 to 3 wt.-% of at least one thickener;
4. 0 to 50 wt.-% of a propellant;
5. 0 to 10 wt.-%, preferably 0.1 to 3 wt.-% of a styling polymer different to 1.), preferably a water soluble non-ionic polymer;
6. 0 to 1 wt.-% of at least one refatter, preferably selected from glycerine and glycerine derivatives;
7. 0 to 30 wt.-% of other customary additives e.g. a silicone component
8. 0.005 to 5 wt.-% of an edelweiss extract according to the present invention,
9. water ad 100 wt.-%

An exemplary conditioner preparation according to the present invention may comprise:
1. 0.05 to 10 wt.-% of a hair polymer
2. 5 to 95 wt.-% of water
3. 5 to 50 wt.-% of surfactant
4. 0 to 5 wt.-% of an additional conditioning agent
5. 0 to 10 wt.-% other customary additives
6. up to 20 wt.-% of an edelweiss extract
all ingredients adding up to 100 wt.-%.

An exemplary styling composition with the compound of the present invention may comprise:
1. 0.1 to 10 wt.-% of at least one hair polymer;
2. 20 to 99 wt.-% water and/or alcohol;
3. 0 to 70 wt.-% of at least one propellant;
4. 0 to 20 wt.-% of customary additives;
5. 0.005 to 5 wt.-% of an edelweiss extract according to the present invention.

An exemplary styling gel with the compound of the present invention may comprise:
1. 0.1 to 10 wt.-% of a hair polymer;
2. 60 to 99.85 wt.-% of water and/or alcohol;
3. 0.05 to 10 wt.-% of a gel former;
4. 0 to 20 wt.-% of other customary additives.
5. 0.005 to 5 wt.-% of an edelweiss extract according to the present invention.

An exemplary hair care composition (spray) with the compound of the present invention may comprise:
1. 0.005 to 5 wt.-% of an edelweiss extract according to the present invention,
2. 30 to 99.5 wt.-%, preferably 40 to 99 wt.-%, of at least one solvent chosen from water, water-miscible solvents and mixtures thereof;
3. 0 to 70 wt.-% of propellant;
4. 0.1 to 10 wt.-% of at least one water-soluble or water-dispersible hair polymer
5. 0 to 0.3 % by weight of at least one water-insoluble silicone;
6. 0 to 0.5 wt.-% of at least one wax, preferably at least one fatty acid amide;
7. customary additives.

Another hair care composition with the compound of the present invention may comprise:
1. 0.05 to 20 wt.-% of at least one hair polymer;
2. 20 to 99.95 wt % of water and/or alcohol;
3. 0 to 79.5 wt.-% of customary additives;
4. 0.005 to 5 wt.-% of an edelweiss extract according to the present invention.

An exemplary composition for aerosol foams with the compound of the present invention may comprise:
1. 0.1 to 10 wt.-% of at least one hair polymer;
2. 55 to 99.8 wt.-% water and/or alcohol;
3. 5 to 20 wt.-% of a propellant;
4. 0.1 to 5 wt.-% of an emulsifier;
5. 0 to 10 wt.-% of customary additives.
6. 0.005 to 5 wt.-% of an edelweiss extract according to the present invention.

An exemplary shampoo preparation with the compound of the present invention may comprise:
1. 0.05 to 10 wt.-% of a hair polymer;
2. 25 to 94.95 wt.-% of water;
3. 5 to 50 wt.-% of surfactant;
4. 0 to 5 wt.-% of an additional conditioning agent;
5. 0 to 10 wt.-% other customary additives.
6. 0.005 to 5 wt.-% of an edelweiss extract according to the present invention,
7. 0 to 5 wt.-% opacifiers and/or pearly gloss-imparting substances

The hair care composition according to the invention can comprise at least a water-soluble or water-dispersible hair polymer. Typical hair polymers for use in the present invention are commercially available polymers for hair care such as hair styling or conditioning polymers such as e.g. copolymers of vinyl acetate and crotonic acid, copolymers of methyl vinyl ether and maleic anhydride, copolymers of acrylic acid or methacrylic acid with other monomers, polyurethanes, N-vinylpyrrolidone and silicone polymers.

The content of the hair polymer is generally from about 0.01 to 10 % by weight, based on the total weight of the composition. Here, it is preferable to use water-soluble or water-dispersible polyurethanes which, if desired, additionally comprise siloxane groups in copolymerized form.

The composition according to the invention can further comprise, at least one water-insoluble silicone, in particular a polydimethylsiloxane, e.g. the Abil^{®} grades from Goldschmidt. The content of the silicone is then generally from about 0.0001 to about 2 % by weight, preferably from about 0.001 to about 1 % by weight, based on the total weight of the composition. Preferred waxes according to the present invention are fatty acid amides, such as, for example, erucamide.

The hair care compositions according to the present invention can, where appropriate, additionally comprise an antifoaming agent, e.g. based on silicone. The amount of antifoaming agent is generally up to 0.001 % by weight, based on the total amount of the composition. The compositions according to the invention have the advantage that, on the one hand, they impart the desired hold to the hair and, on the other hand, the polymers are easy to wash out (redispersible). Generally, a natural appearance and shine is imparted to the hair, even when the hair is by its very nature especially thick and/or dark.

The term alcohol refers to all alcohols usually used in cosmetic compositions such as ethanol, n-propanol, isopropanol.

Other ingredients are cosmetic adjuvants and additives such as propellants, anti-foaming agents, surface active ingredients e.g. tensides, emulsifiers, foam former and solubilisators. The used surface active ingredients may be anionic, cationic, amphoteric or neutral. Further ingredients may be preservatives, antioxidants, perfume oils, lipidic refatters, active and/or caring ingredients such as panthenol, collagen, vitamins, protein hydrolysates, alpha- and beta hydroxyl carbonic acids, stabilisators, pH regulators, opacifiers, colorants, dyes, gel formers, salts, moisturizers, complex formers, viscosity regulators or light screening agents without being limited thereto.

In order to obtain certain properties the hair care compositions may additionally comprise conditioning compounds based on silicone such as polyalkylsiloxane, polyarylsiloxane, polyarylalkylsiloxane, silicone resins, polyethersiloxane or dimethicone copolyole (CTFA) and amino functionalized silicone compounds such as amodimethicone (CTFA), GP 4 Silicone fluid^{®} and GP 7100^{®} (Genesee), Q2 8220^{®} (Dow Corning), AFL 40^{®} (Union Carbide) or polymers as disclosed in EP-B 852 488. Other suitable ingredients comprise silicone propfpolymers having a polymeric silicone backbone and non-silicone containing side chains or a non silicone containing polymeric backbone and silicone side chains such as Luviflex^{®} Silk or polymers disclosed in EP 0 852 488.

Typical propellants for hair sprays or aerosol foams may be used. Preferred are mixtures of propane/ butane, pentane, dimethylether, 1,1-difluoroethane (HFC-152a), carbon dioxide, nitrogen or compressed air.

All emulsifiers for aerosol foams or surfactants for shampoo preparations may be conventionally used non-ionic, cationic, anionic or amphoteric emulsifiers/surfactants.

Examples of non-ionic emulsifiers are (INCl-nomenclature) Laureths, e.g. Laureth-4; Ceteths, e.g. Ceteth-1, polyethyleneglycolcetylether; ceteareths, e.g. ceteareth-25, polyglycol fatty acid glycerides, hydroxylated lecithins, lactyl esters of fatty acids, alkylpolyglycosides. Examples of non-ionic surfactants are e.g. reaction products of aliphatic alcohols or alkylphenols with 6 to 20 C-Atoms of a linear or branched alkyl chain with ethyleneoxide and/or propyleneoxide. The amount of alkyleneoxide is about 6 to 60 mol to one mol alcohol. Furthermore alkylaminoxide, mono- or dialkylalkanolamide, fatty esters of polyethylene glycols, alkylpolyglycosides or sorbitan ester are suitable for the incorporation of hair care compositions according to the invention.

Examples of cationic emulsifiers/surfactants are quaternised ammonium compounds e.g. cetyltrimethylammoniumchloride or bromide (INCl: cetrimoniumchloride or bromide), stearyl benzyl dimethyl ammonium chloride, distearyldimethylammonium chloride, stearamidopropyldimethylamine, hydroxyethylcetyldimonium phosphate (INCl: Quaternium-44), Luviquat^{®} Mono LS (INCl: Cocotrimoniummethosulfate), poly(oxy-1,2-ethandiyl), (octadecylnitrilio) tri-2, 1-Ethandiyl) tris-(hydroxy)-phosphate (INCl Quaternium-52). Furthermore, cationic guar derivatives such as guarhydroxypropyltrimoniumchloride (INCl) may be used in conditioner/shampoo preparations.

Anionic emulsifiers/surfactants can be selected from alkylsulfate, alkylethersulfate, alkylsulfonate, alkylarylsulfonate, alkylsuccinate, alkylsulfosuccinate, N-alkylsarkosinate, acyltaurate, acylisethionate, alkylphosphate, alkyletherphosphate, alkylethercarboxylate, alpha-olefinsulfonate, especially the alkali-und earth alkali salts, e.g. sodium, potassium, magnesium, calcium, as well as ammonium- and triethanol amine-salts. The alkylethersulfate, alkyletherphosphate and alkylethercarboxylate may comprise between 1 to 10 ethyleneoxide or propyleneoxide units, preferably 1 to 3 ethyleneoxide-units per molecule.

Suitable anionic surfactants are e.g. sodium laurysulfate, ammonium laury sulfate, sodium laurylethersulfate, ammonium laurylethersulfate, sodium lauroylsarkonisate, sodiumoleylsuccinate, ammonium laurylsulfosuccinate, sodium dodecylbenzolsulfonate, triethanolamidodecylbenzolsulfonate.

Suitable amphoteric surfactants are e.g. alkylbetaine, alkylamidopropylbetaine, alkylsulfobetaine, alkylglycinate, alkylcarboxyglycinate, alkylamphoacetate or propionate, alkylamphodiacetate or dipropionate such as cocodimethylsulfopropylbetaine, laurylbetaine, cocamidopropylbetaine or sodium cocamphopropionate.

As gel formers, all typical cosmetic gel formers can be used such as slightly cross linked polyacrylic acid e.g. Carbomer (INCl), cellulose derivatives, polysaccarides e.g. xanthan gum, caprylic/ capric triglyceride (INCl), sodiumacrylate-copolymers, polyquaternium-32 (and) paraffinum liquidum (INCl), sodiumacrylate-copolymers (and) paraffinum liquidum (INCl) (and) PPG-1 trideceth-6, polyquaternium-37 (and) propyleneglycoldicapratdicarylate (and) PPG-1 trideceth-6, polyquaternium-7, polyquaternium-44.

In order to provide the formulation a pearlescent appearance or to give the impression of a richer or creamier product, the hair care composition may additionally comprise opacifiers and/or pearly gloss-imparting substances, such as soaps or salts of carboxylic acids, cationics including cationic polymers, dimethicone (INCl) or amodimethicone (INCl).

Other customary additives are for example long chain fatty alcohols such as cetyl alcohol, stearyl alcohol, cetylstearyl alcohol, dimethylstearamine. Furthermore the hair care composition may contain lipids such as dimethicone, amodimethicone, mineral oil, or silicon derivatives such as Dimethicone Copolyol.

In another embodiment, the present invention also relates to a dual-vial packaging system as described in WO2008049443 containing the edelweiss extract as a dried powder in a compartment, and a cosmetically acceptable carrier in the other compartment.

The following Examples are illustrative but not limitative of the invention.

### Examples

### Example 1: Assessment of the effect of an edelweiss extract on gene expression in cultured human hair follicles.

### 1- Material and methods:

A) Test material and preparation: Edelweiss extract (ALPAFLOR^{®} EDELWEISS from DSM) at a concentration of 5 wt-% dry residue was prepared as a stock solution in 60% ethanol. The stock solution was diluted into the cell culture media to the final test concentration.

### D) Hair follicles:

Hair follicles microdissected from a human skin fragment (plastic surgery, face lift, male donor, 54 years old)
Culture conditions: 37°C, 5% CO₂
Culture medium: E William's medium supplemented with penicillin 50U/ml, streptomycin 50ug/ml, L-glutamine 2mM, insulin 10ug/ml, hydrocortisone 0.004ug/ml
13 high quality hair bulbs per condition were cultured for 72 hours. At the end of incubation hair bulbs were immediately frozen in liquid nitrogen and stored at -80°C for RNA extraction.

### E) Analysis of Gene Expression by quantitative PCR technology as PCR array:

The expression of a selection of genes was analysed using RT-qPCR method on mRNA extracted from the cell monolayers for each treatment. Before RNA extraction the replicas were pooled. The analysis of gene expression was then done in duplicates (n=2).
Reverse transcription: Total RNA was extracted from each sample using TriPure solution reagent according to the supplier's advices. The amount and quality of RNA were evaluated using a lab-on-a-chip Bioanalyzer (Agilent Technologies). Potential contaminant traces of genomic DNA were removed using the DNAfree system (Ambion). The amount of the RNA was evaluated using Nanovue (GE Healthcare). The reverse-transcription of mRNA was conducted in presence of oligo(dT) and Superscript II reverse-transcriptase (Invitrogen). Nanovue was also used for quantification of cDNA and DNA concentration adjustment.
Quantitative PCR: The PCRs (Polymerase Chain Reactions) were performed using the Light Cycler system (Roche Molecular Systems Inc.) according to the supplier's instructions. This system allows rapid and powerful PCRs after determining analysis conditions of the test primers. The reaction mix contains diluted DNA, primers forward and reverse, reagent mix containing taq DNA polymerase, SYBR Green I and MgCl2. The incorporation of fluorescence in amplified DNA was continuously measured during the PCR cycles. This resulted in a fluorescence intensity versus PCR cycle plot allowing the evaluation of a relative expression (RE) value for each gene. The value selected for RE calculations is the output point (Ct) of the fluorescence curve. For the considered gene the higher the cycle number the lower is the mRNA quantity. The RE value was expressed in arbitrary units (AU) according to the formula: (1/2number of cycles) x 106. For additional control we also quantified the expression level of three housekeeping genes namely Ribosomal protein S28, Glyceraldehyde-3-phosphate dehydrogenase and beta-Actin in both untreated control cells and treated cells. The mean relative expression level of the three housekeeping genes was set to 100% and the variation in expression level of individual three genes was within less than 100 ± 50%.

### 2- Results:

The edelweiss extract modulated the gene expression of following key markers as summarized in Table 1:
Keratins:
   KRT34, 82, and 85 are located in the hair shaft's cuticle and cortex. They belong to the structural proteins making up the hair shaft. For KRT85 it is known that a mutation in the gene leads to hypotrichosis (hair fall). There is a case report: Shimomura Y, Wajid M,Kurban M, Sato N, Christiano AM. Mutations in the keratin 85 (KRT85/hHb5) gene underlie pure hair and nail ectodermal dysplasia. J Invest Dermatol. 2010 Mar;130(3):892-5.
Growth factor receptor:
   FGFR2: Receptor for fibroblast growth factor. This gene is implicated in hair thickness (see e.g. Fujimoto A, Nishida N, Kimura R, Miyagawa T, Yuliwulandari R, Batubara L, Mustofa MS, Samakkarn U, Settheetham-Ishida W, Ishida T, Morishita Y, Tsunoda T, Tokunaga K, Ohashi J. FGFR2 is associated with hair thickness in Asian populations. J Hum Genet. 2009 Aug;54(8):461-5).

**Table 1: Gene expression results in microdissected cultured human hair follicles expressed as percentage of expression compared to control:**

| **Genes** | **Edelweiss (EW)** | |
|---|---|---|
| | 3.33 µg/ml | 10 µg/ml |
| | % Control | % Control |
| **KRT82** | 268 | 173 |
| **KRT85** | 151 | 147 |
| **KRT34** | 224 | 151 |
| **FGFR2** | 126 | 139 |

## Claims

1. The use of a topical composition comprising an edelweiss extract for lessening hair loss, restoring hair growth after the onset of baldness has occurred, increasing the thickness of hair, counteracting age-associated hair thinning, preventing premature hair loss, or delaying the onset or severity of age-associated hair loss and thinning.

2. The use according to claim 1, wherein the topical composition is a hair care composition

3. The use according to any of claims 1 or 2, wherein the edelweiss extract is combined with the use of at least one additional active substance selected from the group consisting of antioxidants, light screening agents, colorants.

4. The use according to any of claims 1 to 3, wherein the composition comprises 0.0001 wt.% to 10 wt.% edelweiss extract based on the total weight of the topical composition.

5. The use according to any of claims 2 to 4, wherein the hair care composition is a hair tonic, a conditioner, a treatment, or a styling gel.

6. The use according to any of claims 1 to 5, wherein the topical composition is applied at least twice a day.

7. Method for stimulating hair growth, comprising the steps of applying to skin having hair, for a sufficient time, a topical composition comprising an effective amount of an edelweiss extract, and observing the results.

8. The method as in claim 7, wherein the effective amount of the edelweiss extract is selected in the range of 0.0001 to 10 weight-% based on the total weight of the topical composition.

9. The method according to any one of claims 7 to 8, wherein the topical composition is a hair care composition.

10. The method as in claim 9, wherein the hair care composition is a hair tonic, a conditioner, a shampoo, or a styling gel.

11. The method according to any one of claims 7 to 10, wherein the topical composition further comprises at least one additional active substance selected from the group consisting of antioxidants, light screening agents, and colorants.
